# EUROPEAN PATENT APPLICATION

(11) **EP 0 568 383 A1**
(43) Date of publication of application: **03.11.1993**
(21) Application number: 93303400.1
(22) Date of filing: 30.04.1993
(51) Int. Cl.: A61B 17/34, A61M 39/00

(54) **Sealing mechnism for surgical trocar**

(30) Priority: 01.05.1992 US 877903
(71) Applicant: ETHICON INC., Somerville New Jersey 08876 (US)
(72) Inventor: Failla, Stephen J., Cincinnati, Ohio 45241 (US)
(74) Representative: Fisher, Adrian John

(57) **Abstract**

A trocar seal is provided which has a plurality of gasketing mechanisms (55) placed across its seal housing inner diameter. These gasketing mechanisms (55) are positioned in angular relationship to one another so that they cover the entire seal housing opening ; however, the gasketing mechanisms (55) are resilient and act as both a seal and as a valving mechanism for use within the trocar. They allow surgical instruments of varying dimensions to be placed within the cannula (30), either singly or simultaneously.

## Description

### Field of the Invention

Generally, this invention relates to surgical trocars. Specifically, this invention relates to seals for surgical trocars. Most specifically, this invention relates to seals for surgical trocars which allow the user to introduce instruments of many sizes through the cannula of the trocar without compromising the sealing capabilities of the seal during insertion of these instruments into the cannula.

### Background of the Invention

Generally, surgical trocars are instruments which contain three mechanisms. There is an obturator for cutting the abdominal wall; there is also a cannula, which is inserted around the obturator into the abdomen. Finally, there is a shield, which is generally spring-loaded and capable of covering the obturator during use within the abdominal wall.

After insertion of the trocar mechanism into the abdominal wall, the handle of the mechanism is separated into two parts. The handle half containing the trocar cannula remains inserted within the body. The half containing the obturator is removed, along with the safety shield.

In the handle half containing the trocar cannula, there generally has been used various valving and sealing mechanisms to allow insufflation of the abdomen during use of the trocar. Generally, these valving mechanisms have been comprised of toilet-seat or "flapper" valves, or other such mechanisms which either remain fully open or fully closed. Also, external to these valve mechanisms there are placed seals, which are generally elastomeric membranes having concentric circular openings, slightly smaller in diameter than the surgical instrument for which the cannula is sized. This seal promotes gaseous entrapment whenever the valve is opened by passage of an instrument. In this way, the surgeon is crudely capable of using an instrument of generally the same size of the trocar cannula without the worry of desufflation of the abdomen through the cannula.

However, as these trocars are of varying dimensions, that is between 3mm innerdiameterand 18mm inner diameter, and it is envisioned that even larger dimensions may be possible, it is of concern that the dimensions of the seal inner diameter becomes a limiting variable for the use of certain smaller diameter instruments within the trocar cannula. Design attempts have been tried whereby the trocar is capable of being reduced in effective diameter from a larger inner diameter to a smaller inner diameter. Yet, this solution also has the drawback of necessarily reducing the effective size of the cannula so that only certain instruments can be used within this cannula.

Therefore, it would be desirable to create a seal on a trocar which allows the user to capably insufflate the abdomen, and yet also very readily enjoy the capabilities of inserting surgical instruments of varying sizes within the cannula without any reducer seals. If the valving mechanism were capable of emplacement within the cannula handle so that it readily accepts surgical instruments of varying sizes and seals around them virtually instantaneously, then the user could insert and remove the surgical instruments without desufflating the abdomen, which problem was previously encountered. Also, with use of such a sealing mechanism, it would be further be able to create a trocar seal which could be used in virtually any cannula without the dimensional needs of variously sized trocar cannulas. Finally, it would be desirable to have a trocar seal which permits insufflation, and yet allows the easy passage therethrough of instruments with variable diameters, such as circular staplers.

### Summary of the Invention

Therefore, it is an object of the invention to provide a trocar with a variable diameter sealing mechanism in its cannula handle.

It is another object of the invention to provide a trocar with a seal which allows variable openings in the trocar cannula.

It is another object of the invention to provide a seal which allows for the user to have the capability of inserting instruments of varying diametric size at the same time through the cannula, orone instrument with varying diameters, such as a circular staplers.

It is yet another object of the invention to provide a sealing mechanism which negates the need for typical flapper type valves because of its enhanced sealing functions.

Finally, it is an object of the invention to provide a trocar sealing mechanism which allows the user to forego the necessity of providing reducing instruments in order to permit a large diameter trocar because to seal effectively around smaller instrument shafts.

These and other objects of the invention are accomplished in a trocar seal which provides a plurality of gaskets formed from sheet rubber or any other resilient material which extend across more than about half the diameter of the cannula opening. Each of these rubber gaskets is splayed across the opening, sometimes typically in a spiral type fashion. Thus, the diameter of the cannula is covered with the use of very few such gasketing type devices placed across its opening.

With these gasketing materials placed across the cannula opening, one is capable of readily placing a surgical instrument of any acceptable dimension within the trocar cannula. This is done by centralizing the surgical instrument as it is placed within the cannula. The remainder of the trocar seal is sealed about the surgical instrument. Both during and after placement sealing takes place around the instrument. Thus, once the instrument is fully placed within the cannula, the sealing material resiliently and reliably holds the insufflated abdominal pressure at the sealing mechanism, with the surgical instrument securely inserted through the cannula.

This invention allows the user to capably insert many such surgical instruments of varying diameters within a large diameter trocar without the need for of a flapper type valve, or any other device, such as a seal reducer type mechanism.

These and other objects of the invention will be understood from the attached drawings and detail description of the invention which follow.

### Description of the Drawings

Figs. 1, 2 and 3 are close-up and perspective views of a typical trocar with the sealing mechanism of this invention in place;
Fig. 4 is a perspective view of a prior art type surgical trocar valve;
Fig. 5 is an assembly view of a valve of the present invention;
Figs. 6, 7 and 8 are top views of a valve of the invention with an instrument not inserted, and therein;
Fig. 9 is a cross sectional view of the valve of this invention as taken across lines 9-9 of Fig. 6; and
Figs. 10, 11, 12 and 13 are views of alternate embodiments of this invention.
Figure 14 is a cross sectional view of an embodiment of the invention permitting passage of a rectangular cross section instrument; and
Figure 15 is a cross section similar to Figure 9, but showing folded gaskets expanded into gas pockets.

### Detailed Description of the Invention

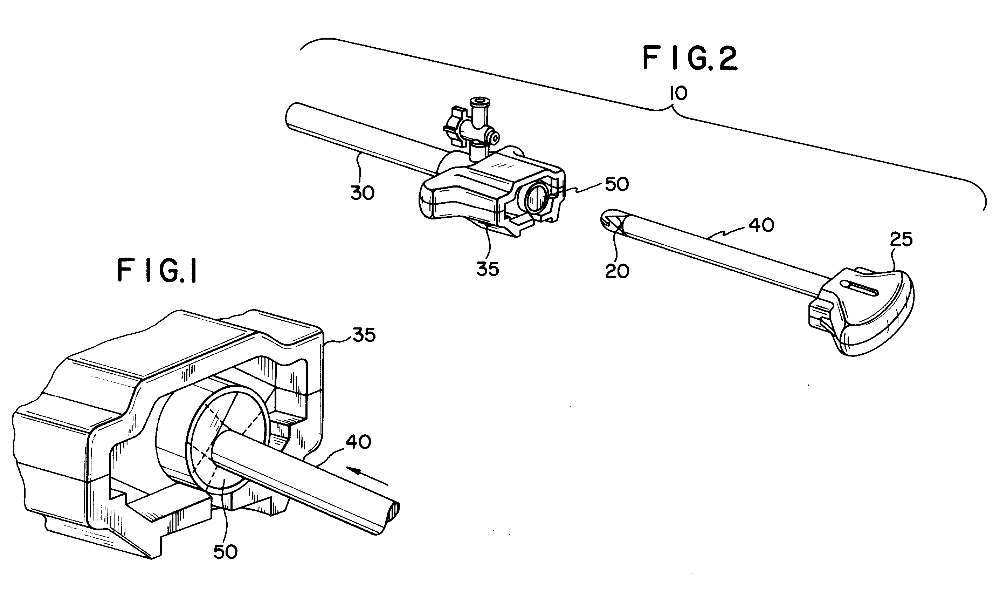

As seen in Figures 1, 2 and 3, a typical surgical trocar 10 contains a obturator 20 or cutting mechanism, held within a cannula 30. Also, there is a spring-loaded shield 40 which covers the obturator 20 after it has been inserted within the abdominal cavity. The obturator is held along an obturator handle 25. The cannula 30 is held on the other half of the handle 35, commonly referred to as the cannula handle.

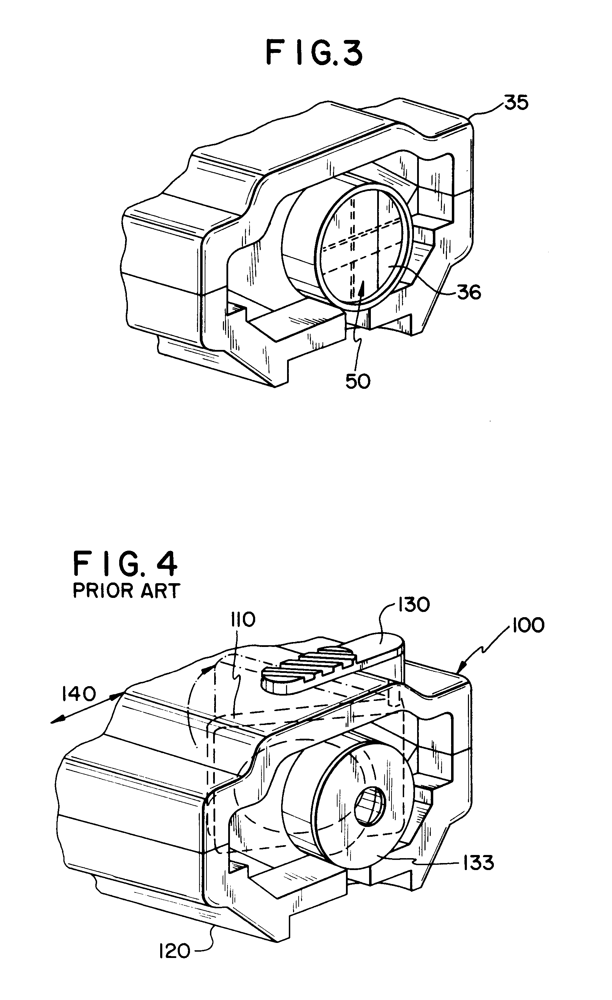

As seen in Fig. 4, in prior art trocars 100 there were contained valving mechanisms, which generally comprised flapper type valves 110. Thus, the flapper valve 110 was held within the cannula handle 120, and when an instrument was inserted, the flapper valve 110 is pivoted open to accommodate the instrument as indicated by lever 130. A diaphragm seal 133 provides a gas-tight fit with the instrument shaft, preventing leaks when valve 110 is open. One of the constraints on such a mechanism would be that the outside diameter of the instrument inserted must necessarily be smaller than the inner diameter of the cannula 30 and only slightly larger than the inside diameter of seal 133, or pressurized gas used for insufflation would escape through the flapper valve 110. The valve 110 could not close at all about the endoscopic instrument, unless this diametric constraint was met.

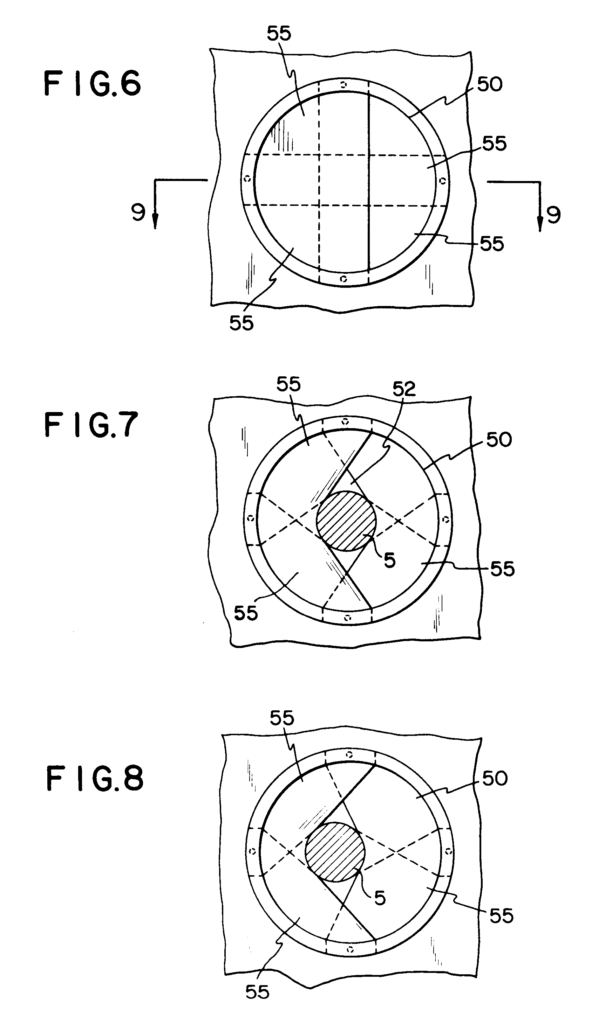

As seen in Figs. 3, 5, 6, 7, 8 and 9, these worries are obviated by the present cannula seal 50 as described by this invention. As seen in Figs. 3, 6 and 9, the seal 50 of this invention is comprised of a number of resilient gaskets 55 placed across the diameter 32 of the seal ring 37. These gaskets 55 cover roughly one half to three-fourths of the opening formed by the inner diameter 32 of seal ring 37. The gaskets 55 are formed from sheets of a resilient, yet strong, sealing material, such as rubber. These are sealed such as by clamping heat sealing or tacking to the outer diameter 34 of the trocar cannula 30 or between seal housing 39 and seal ring 37, held within the cannula handle 35. These gasketing materials, therefore, each cover about one half of the inside diameter 32 of seal ring 37. Desirably, it is useful to cover a little more than one half, generally 70% of the cannula opening 32.

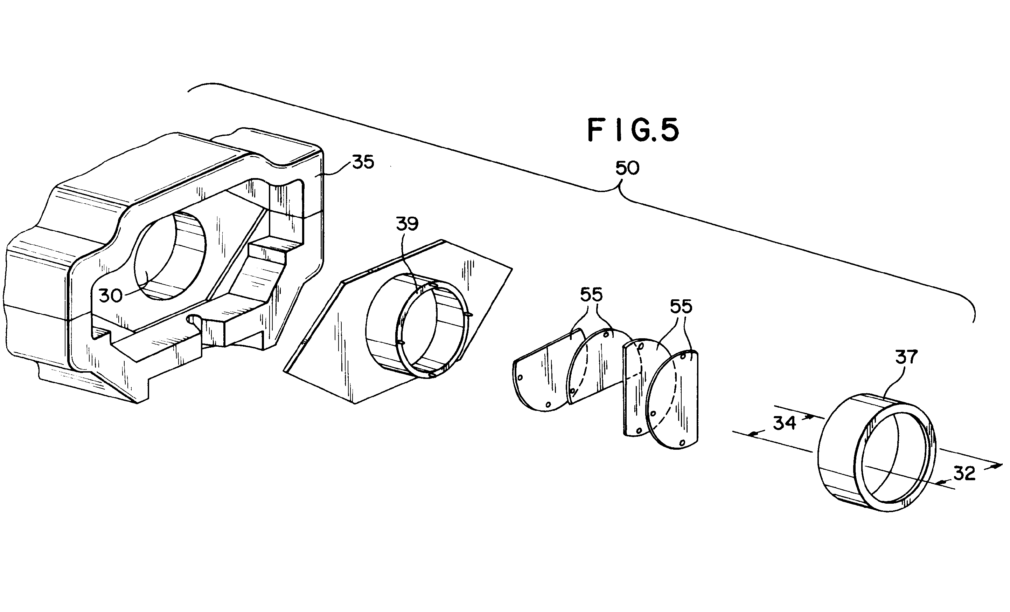

As can be seen from Figs. 5, 7, 8 and 11, each of these gasketing materials 55 is placed across the opening at an angularly displaced relationship with each of the other gasketing materials 55. Thus, depending upon the angular spacing, it can very quickly be seen that the entire opening 32 of the seal ring 37 will be covered with only a very few number of such gaskets 55. However, it has been found that generally 4 to 12 such gaskets are the most effective in operating this invention. When these gaskets are arranged in such spiral, they form a resilient and adaptable seal across the seal surface 36.

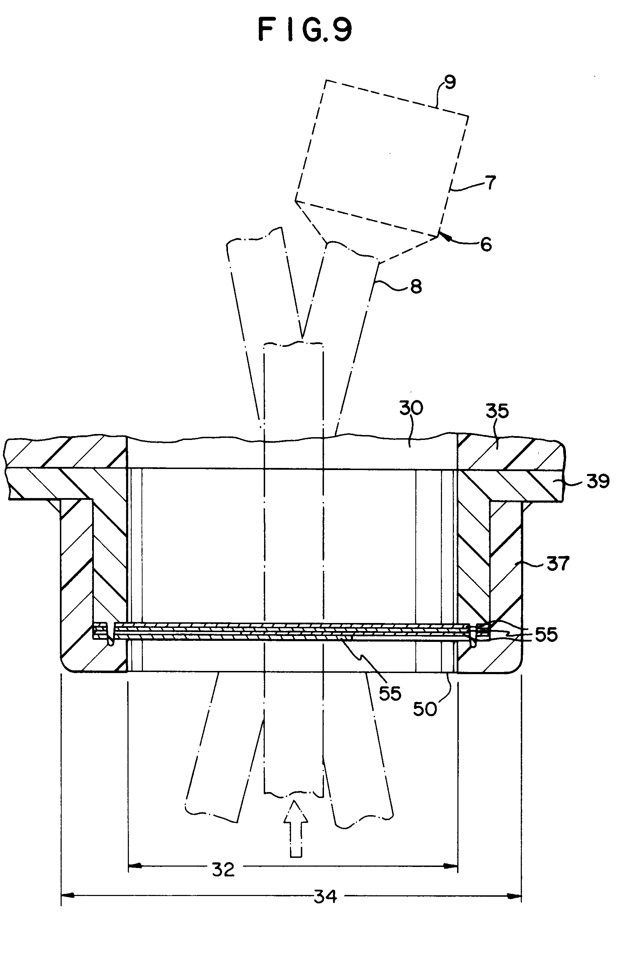

As can be seen in Figs. 7 and 8, a number of surgical instruments can be inserted within the surgical cannula 30. These instruments may be inserted within the seal 50 in the following way: Generally, the user must place the instruments eccentrically within the seal 50 and apply a generally orbiting motion. Such a motion allows the user to "find" the center 52 of the opening, by progressively passing each of the gasket 55 layers. This should happen after and between 1 and 3 orbits, dependent on the number of gasket layers 55. In this way, there is less chance of ripping the gasketing material 55, or being prevented from insertion of the surgical instrument into the cannula.

Once the instrument has been inserted past the seal 50, it may be operated in the typical method for use of surgical instruments within trocars. However, it has been found that with this type seal the user is capable of placing two or more instruments simultaneously within this seal 50. This is especially true because the user is capable of varying the seal opening without compromising the sealing capabilities of the mechanism.

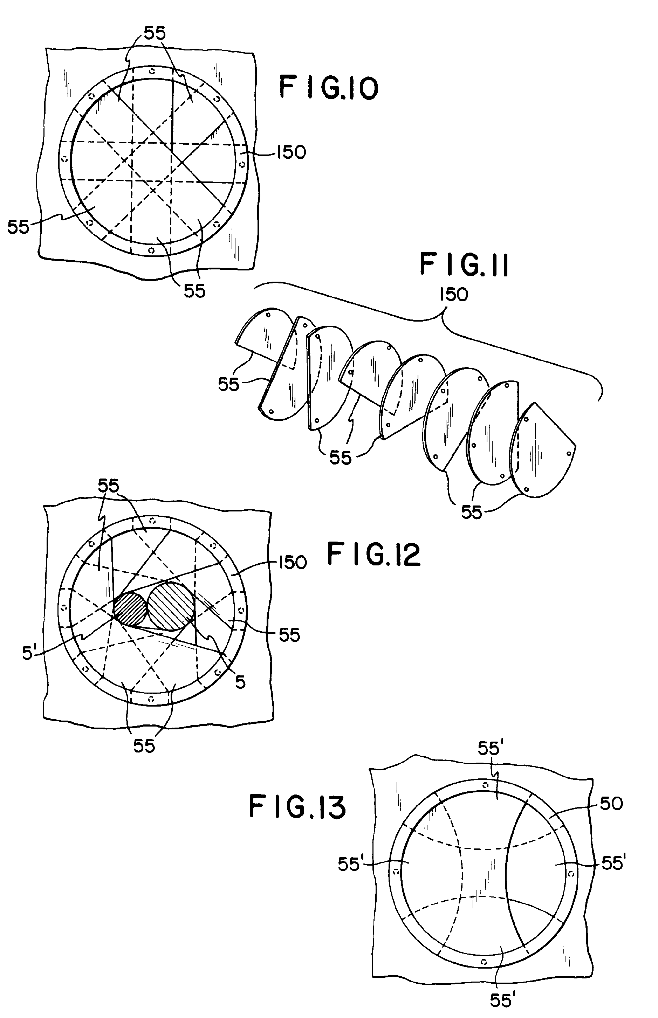

As can be seen in Figure 12, with a number of instruments inserted, these instruments can be capably placed next to the existing instruments and "snaked" down the cannula or trocar tube 30. Alternately, these instruments can be removed, again without compromising the usefulness of this seal 50.

Also, it will be seen in Figures 8 and 9, that the instruments, as inserted, if smaller in outer diameter than the inner diameter of the trocar cannula 30, can be moved across the seal ring opening 32 with the seal remaining intact. This is because as the surgical instrument is moved in any direction, parts of the gasketing material 55 are relaxed, while other parts are put under tension. Thus, the sealing mechanism "follows" the surgical instrument across the cannula opening. In this way, this mechanism achieves a far greater advantage than the previously used flapper valve mechanisms. This "following" is better seen in Figures 8 and 9.

As seen in Figs. 10, 11, and 12, there is described a seal arrangement 150 using eight such gaskets 55. It has been found that this arrangement also quite readily accommodates surgical instruments 5, 5', as better seen in Fig. 12. This configuration, however, is capable of more tightly holding such instruments in the center of the seal arrangement 150, as more gaskets 55 enhance sealing effect. This may be advantageous in order to insure proper placement of the instruments 5, 5' within the abdominal cavity.

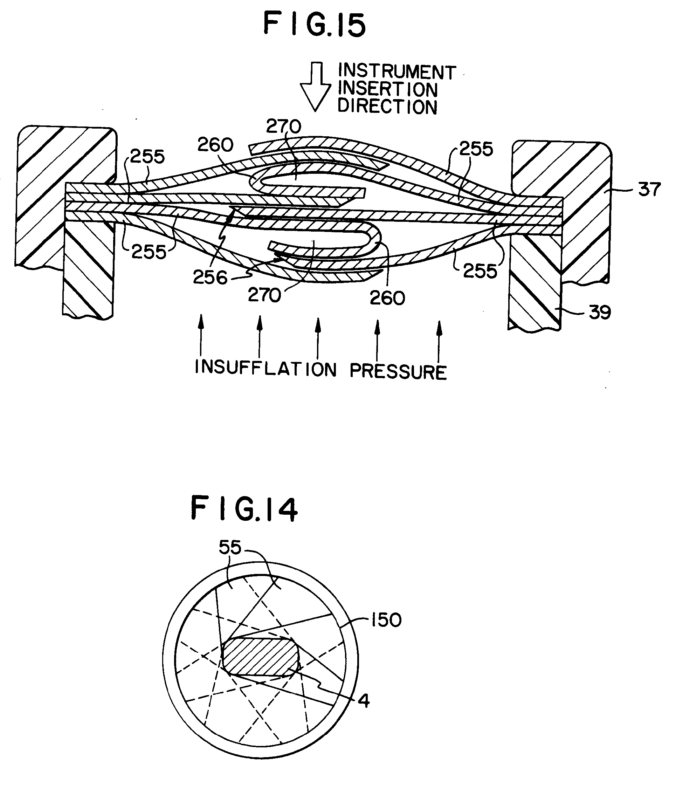

As seen in Fig. 13, the seal 50 has been configured with "scalloped" edges 55'. These edges have been "scalloped" in order to more readily provide an increased wrap arc for the shaft of a surgical instrument in the center of the cannula channel. This may be desirable, if the instrument is quite large in relation to the opening.

As seen in Fig. 9, a surgical instrument 6, having a large diameter head 7 with a flat end 2 and a smaller diameter shaft 8 has been inserted through the seal 50. This instrument configuration is typical of commonly used circular staplers. Instruments 6 of this sort are readily insertable and removable through this seal 50, and the seal accommodates to sealing over both the head and the shaft.

As seen in Fig. 14 a surgical instrument 4, having a non-circular cross section is capably inserted through and sealed by seal 50.

Fig. 15 uses the clamping configuration of Fig. 9, but orients some of the internal gaskets 255, in a folded position. This enhances sealing as the folds 260 entrap any leaving gas and expand into pocket 270. These pockets 270 force the gaskets 255 axially apart, thus sealing them more tightly together. These pockets 270 would help seal the multiple instrument array shown in Fig. 12. There are also seen bevels 256 on the edges of some of the internal gaskets. Although these gaskets are thin [6-15 mils], the bevel is a desirable enhancement for reducing gas leakage.

For all these reasons, therefore, it is seen that this valving mechanism provides a reliable, easy to make, and capable improvement over existing mechanisms. However, it is to be realized thatthe invention is to be understood from the attached claims and their equivalents.

## Claims

1. A surgical trocar including:
an obturator;
a tubularcannula having an innerdiameter into which is insertable said obturator,
said cannula being attached to a cannula handle, said handle being adapted to contain a sealing mechanism; and
a sealing mechanism in said cannula handle, wherein said sealing mechanism is capable of sealing about tools of any size smaller than said inner diameter.

2. A surgical trocar including:
an obturator;
a cannula handle;
a tubular cannula attached to said cannula handle having an inner diameter into which is insertable said obturator;
said cannula handle being adapted to contain a sealing mechanism; and
a sealing mechanism attached to said cannula handle having an inner diameter wherein said sealing mechanism is adapted to accept and seal about surgical instruments inserted into said cannula, independent of whether said instruments are inserted within the center of said sealing mechanism inner diameter.

3. A surgical trocar including:
an obturator;
a cannula handle having an inner diameter into which is insertable said obturator,
said cannula being adapted to contain a sealing mechanism; and
a sealing mechanism with an inner diameter, wherein said sealing mechanism is capable of sealing about more than one tool simultaneously inserted into said cannula.

4. The surgical trocar of any preceding claim wherein said sealing mechanism comprises a plurality of resilient gaskets, said gaskets being stretched across said inner diameter of said sealing mechanism to cover a portion of said inner diameter, and said gaskets being placed in a spaced apart angular relationship with respect to one another.

5. The surgical trocar of claim 4 wherein there are at least eight said gaskets.

6. The surgical trocar of claim 4 wherein at least one of said gaskets covers at least one-half of said inner diameter of said cannula.

7. The surgical trocar of claim 4 wherein said cannula is at least 10mm in diameter.

8. The surgical trocar of claim 4 wherein said gaskets are connected to said cannula within said cannula handle.

9. The surgical trocar of claim 4 wherein said gaskets are adapted to open to allow tools of variable size diameters therethrough.

10. The trocar of claim 4 wherein at least one of said gaskets is folded when placed across said inner diameter.

11. The trocar of claim 4 wherein at least one of said gaskets has a bevelled edge.
